(19) Europäisches Patentamt / European Patent Office / Office européen des brevets

(11) **EP 3 220 131 B1**

(12) **FASCICULE DE BREVET EUROPEEN**

(45) Date de publication et mention de la délivrance du brevet:
**21.09.2022 Bulletin 2022/38**

(21) Numéro de dépôt: **17161401.9**

(22) Date de dépôt: **16.03.2017**

(51) Classification Internationale des Brevets (IPC):
**G01N 21/17** *(2006.01)*  **G01N 21/27** *(2006.01)*
**G01N 21/59** *(2006.01)*  **G01N 21/78** *(2006.01)*
**G01N 15/06** *(2006.01)*  **G01N 15/14** *(2006.01)*
**G01N 33/66** *(2006.01)*  **G01N 33/84** *(2006.01)*
**C12Q 1/54** *(2006.01)*  **G01N 1/40** *(2006.01)*
**G01N 21/80** *(2006.01)*  *G01N 15/00* *(2006.01)*

(52) Classification Coopérative des Brevets (CPC):
**G01N 21/1717; G01N 15/06; G01N 21/59;**
**G01N 21/78; G01N 33/66; G01N 33/84;**
G01N 21/274; G01N 21/80; G01N 2001/4094;
G01N 2015/0065; G01N 2015/0687;
G01N 2015/0693; G01N 2021/1729

(54) **PROCÉDÉ ET DISPOSITIF DE CARACTÉRISATION D'UN ÉCHANTILLON LIQUIDE COMPORTANT DES PARTICULES**

VERFAHREN UND VORRICHTUNG ZUR CHARAKTERISIERUNG EINER FLÜSSIGEN, PARTIKEL ENTHALTENDEN PROBE

METHOD AND DEVICE FOR CHARACTERISING A LIQUID SAMPLE COMPRISING PARTICLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.03.2016 FR 1652263**

(43) Date de publication de la demande:
**20.09.2017 Bulletin 2017/38**

(73) Titulaire: **COMMISSARIAT À L'ÉNERGIE ATOMIQUE ET AUX ÉNERGIES ALTERNATIVES**
**75015 Paris (FR)**

(72) Inventeurs:
• **AUBERT, Vivian**
  **38000 GRENOBLE (FR)**
• **CUBIZOLLES, Myriam-Laure**
  **38700 CORENC (FR)**
• **POULAIN, Cédric**
  **38000 GRENOBLE (FR)**
• **HUET, Maxime**
  **38000 GRENOBLE (FR)**
• **RABAUD, David**
  **38000 GRENOBLE (FR)**

(74) Mandataire: **INNOV-GROUP**
  **310, avenue Berthelot**
  **69372 Lyon Cedex 08 (FR)**

(56) Documents cités:
**EP-B1- 1 875 203      WO-A1-90/05008**
**WO-A1-94/20833      WO-A1-2008/060235**
**WO-A1-2011/006525**

**Description**

# DOMAINE TECHNIQUE

Le domaine technique de l'invention est l'analyse optique d'un liquide comportant des particules, l'analyse optique étant couplée à une segmentation du liquide en une phase enrichie en particules et une phase appauvrie en particules.

# ART ANTERIEUR

La mesure de la glycémie est une mesure couramment pratiquée à l'aide de dispositifs de mesures portables, utilisables à domicile, dans des applications dénommées « Point of Care », terme anglais signifiant au chevet du patient, ou chez le patient. Cette mesure peut être réalisée par une méthode optique, en mettant en œuvre une réaction enzymatique entraînant la formation d'un indicateur coloré, par exemple à partir d'un sel de tétrazolium. Un échantillon sanguin est alors disposé entre une source de lumière et un photodétecteur, ce dernier mesurant une intensité de la lumière transmise par l'échantillon. Cependant, le taux d'hématocrite dans le sang peut avoir une influence sur la mesure. En effet, des particules sanguines, notamment les globules rouges, diffusent et/ou absorbent la lumière traversant l'échantillon, et il est nécessaire de prendre en compte cette perturbation.

Une première option, pour s'affranchir de l'effet des particules, est de procéder préalablement à la mesure, à une hémolyse. C'est ce qui est décrit dans le brevet US5866349. Dans ce brevet, on décrit une méthode optique pour déterminer la concentration de glucose dans du sang total. Après une étape d'hémolyse, la méthode met en œuvre la réaction enzymatique précédemment évoquée. Cependant, l'étape d'hémolyse peut présenter certains inconvénients : d'une part, elle constitue une étape additionnelle, supposant l'ajout d'un réactif de lyse ainsi qu'un temps d'attente pour que la quantité de particules lysées soit suffisante. D'autre part, comme indiqué dans la demande EP1875203, la lyse de particule peut libérer, dans le plasma sanguin, des composants intracellulaires susceptibles de réagir avec l'indicateur coloré. Ainsi, l'étape de lyse ajoute de la complexité et peut impacter la précision de la mesure.

Des alternatives à l'hémolyse existent. Par exemple, le brevet EP1875203 décrit un dispositif permettant d'estimer une quantité de glucose dans un échantillon sanguin, sans mettre en œuvre l'hémolyse. Le principe de mesure repose également sur la formation d'un indicateur coloré issu de la réduction d'un sel de tétrazolium. L'échantillon sanguin est couplé au photodétecteur par deux lentilles, disposées successivement entre l'échantillon et le photodétecteur. Ces lentilles permettent d'augmenter le signal collecté par le photodétecteur. Le photodétecteur peut être un photodétecteur matriciel, de type CCD. Deux sources de lumière sont utilisées, l'une émettant dans une bande spectrale d'absorption de l'indicateur coloré, l'autre émettant dans le proche infrarouge. La détection du rayonnement lumineux transmis par l'échantillon, illuminé dans l'infrarouge, permet de déterminer un taux d'hématocrite ; la détection du rayonnement lumineux transmis par l'échantillon dans la bande spectrale d'absorption de l'indicateur permet d'estimer une quantité de glucose, cette estimation étant corrigée du taux d'hématocrite préalablement déterminé. Mais la mise en œuvre d'un système optique complexe, basé sur deux lentilles hémisphériques, nuit à la compacité du dispositif, ainsi qu'à son coût.

Plus généralement, au-delà du sang, l'analyse d'un liquide comportant des particules peut poser des problèmes liés à la présence desdites particules. Des procédés connus ont recours à une centrifugation de l'échantillon pour pouvoir effectuer une séparation efficace des particules. Mais la centrifugation est une technique difficilement intégrable dans un dispositif de type point of care Elle suppose l'utilisation de pièces mobiles et des actuateurs puissants. Elle peut également entraîner une lyse de certaines particules.

Certains documents décrivent des procédés permettant de caractériser des particules en suspension dans un échantillon liquide en se basant sur la propagation d'une onde acoustique dans l'échantillon liquide. Il s'agit par exemple de WO2011/006525, qui décrit un procédé permettant d'estimer la taille ou le nombre de particules. Le document WO94/20833 utilise un principe similaire pour effectuer une séparation de particules en fonction de leur taille.

L'invention proposée adresse ce problème, en proposant une méthode simple, permettant d'obtenir une caractérisation d'un liquide comportant des particules. L'invention peut être mise en œuvre à l'aide d'un dispositif simple et peu couteux, ne nécessitant ni système optique complexe, ni l'illumination successive de l'échantillon par deux longueurs d'onde différentes.

# EXPOSE DE L'INVENTION

Un premier objet de l'invention est un procédé de caractérisation d'un échantillon selon la revendication 1.

Par onde lumineuse transmise, on entend une onde lumineuse résultant de l'illumination de l'échantillon par l'onde lumineuse incidente, après que cette dernière a traversé l'échantillon.

Les termes nœuds et ventres de pression désignent respectivement des zones de l'échantillon dans lesquelles l'amplitude de l'onde de pression acoustique est respectivement minimale et maximale.

Sous l'effet de l'onde acoustique, les particules présentes dans l'échantillon peuvent se concentrer soit au niveau desdits nœuds de pression, soit au niveau des ventres de pression.

Selon un mode de réalisation, la caractérisation de l'échantillon comporte une estimation d'une quantité d'un analyte dans l'échantillon. L'étape c) comporte alors une estimation de la quantité de l'analyte en fonction d'une intensité de l'onde lumineuse détectée par le photodétecteur. Le procédé peut inclure, préalablement à l'étape b), un mélange de l'échantillon avec un réactif apte à modifier une propriété optique de l'échantillon.

Par propriété optique, on entend l'absorption ou de la diffusion de l'échantillon dans une ou plusieurs bandes spectrales. Il peut s'agir d'une couleur de l'échantillon.

Selon un mode de réalisation, le réactif est apte à former un indicateur coloré dans l'échantillon, sous l'effet dudit analyte. La quantité d'indicateur coloré formée dépend généralement de la quantité d'analyte, par selon une fonction croissante de ladite quantité d'analyte. Selon l'invention, la caractérisation comprend une détermination d'une propriété optique du milieu liquide dans lequel baignent les Selon l'invention, le photodétecteur est un capteur d'image, de telle sorte que :

- l'étape b) comprend l'acquisition d'une image de l'onde lumineuse transmise par l'échantillon ;
- l'étape c) comprend une identification, sur ladite image, d'une région d'intérêt correspondant à au moins une partie appauvrie en particules, la caractérisation étant réalisée à partir de la région d'intérêt ainsi identifiée.

Selon une variante, il n'y a pas d'optique de grossissement, entre le capteur d'image et l'échantillon.

Le procédé peut comporter l'une quelconque des caractéristiques suivantes, prises isolément ou en combinaison :

- l'échantillon est maintenu sur un support fluidique, l'application de l'onde acoustique étant réalisée au moyen d'un transducteur électromécanique, notamment piézoélectrique, agissant sur ledit support fluidique de façon à propager l'onde acoustique dans ledit échantillon. Le support fluidique peut être une chambre fluidique, définissant une épaisseur de l'échantillon selon une direction de la propagation de l'onde lumineuse incidente.
- l'onde acoustique appliquée à l'échantillon est une onde stationnaire ;
- l'échantillon comporte un liquide corporel, en particulier du sang ;
- les particules sont des globules rouges.

Un autre objet de l'invention est un dispositif pour caractériser un échantillon, comportant des particules baignant dans un milieu liquide, selon la revendication 11.

Le photodétecteur es un capteur d'image. Le processeur est apte à identifier, sur au moins une image acquise par le capteur d'image, une région d'intérêt correspondant à une partie appauvrie en particules, le processeur étant configuré pour caractériser l'échantillon à partir de la région d'intérêt ainsi identifiée. Selon un mode de réalisation, il n'y a pas d'optique de grossissement, entre le support fluidique et le capteur d'image.

Le support fluidique peut être une chambre fluidique, configurée pour confiner l'échantillon entre la source de lumière et le photodétecteur. Cette chambre fluidique peut notamment définir une épaisseur de l'échantillon, par exemple une épaisseur constante, selon une direction de propagation de l'onde lumineuse incidente.

D'autres avantages et caractéristiques ressortiront plus clairement de la description qui va suivre de modes particuliers de réalisation de l'invention, donnés à titre d'exemples non limitatifs, et représentés aux figures énumérées ci-dessous.

## FIGURES

La figure 1A représente un dispositif selon un mode de réalisation de l'invention, illustrant un échantillon placé dans une chambre fluidique couplée à un transducteur, le transducteur n'étant pas activé. La figure 1B illustre une configuration similaire à la figure 1A, le transducteur étant activé. La comparaison des figures 1A et 1B permet de comprendre l'effet technique lié à l'activation d'un tel transducteur. La figure 1C représente un exemple de chambre de fluidique pouvant accueillir un échantillon.

La figure 1D schématise des conditions géométriques propices à l'établissement d'une onde de pression stationnaire dans une chambre fluidique.

La figure 2 représente une image réalisée avec un dispositif tel que représenté selon la figure 1B, l'échantillon comportant du sang dilué dans un tampon.

Les figures 3A et 3B représentent respectivement des images d'un échantillon dit de référence respectivement sans et avec activation de transducteurs couplés à la chambre fluidique, cette dernière étant similaire à l'exemple présenté sur la figure 1C.

Les figures 4A et 4B représentent des images respectives de deux échantillons de test lors du dosage d'une quantité de glucose, ces images étant obtenues 109 secondes après activation des transducteurs piézoélectriques.

La figure 5A représente l'évolution, en fonction du temps, de l'intensité moyenne des parties appauvries des images de l'échantillon de référence et de deux échantillons de test.

La figure 5B représente l'évolution, en fonction du temps, d'un ratio représentatif de l'intensité moyenne des parties appauvries en particules respectivement des images de l'échantillon de référence et de deux échantillons de test, sur l'intensité moyenne de la partie appauvrie en particules de l'image de l'échantillon de référence.

Les figures 6A, 6B et 6C représentent des images de trois échantillons de test présentant des taux d'hématocrite respectivement égaux à 41%, 25% et 10%.

La figure 7 représente l'évolution, en fonction du temps, l'aire cumulée des surfaces claires des figures 6A, 6B et 6C, ces surfaces claires représentant les parties appauvries de l'échantillon.

La figure 8 est une courbe de calibration obtenue en fonction des essais présentés en lien avec les figures 6A, 6B, 6C et 7. Les figures 6A, 6B, 6C, 7 et 8 ne concernent pas l'invention revendiquée.

La figure 9 illustre un autre mode de réalisation de l'échantillon.

## EXPOSE DE MODES DE REALISATION PARTICULIERS

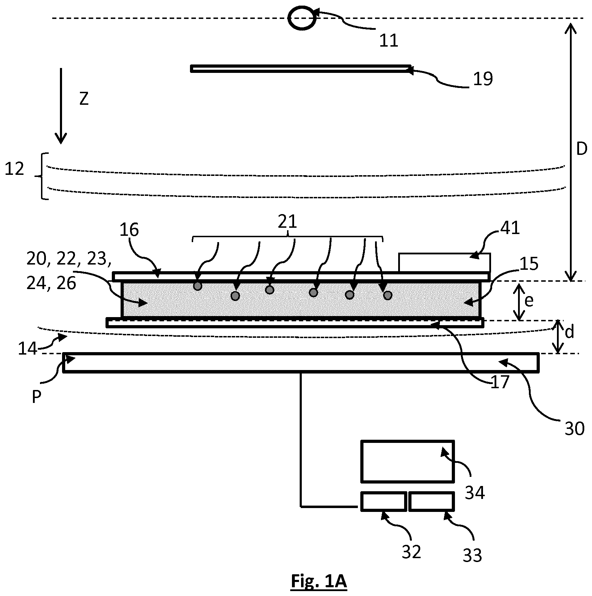

La figure 1A représente un exemple de dispositif objet de l'invention. Une source de lumière 11 est apte à émettre une onde lumineuse 12, dite onde lumineuse incidente, dans une bande spectrale d'illumination, en direction d'un échantillon 20, selon un axe de propagation Z.

L'échantillon 20 comporte un milieu liquide 22 ainsi que des particules 21 baignant dans ce milieu liquide. L'échantillon 20 peut notamment comprendre un liquide corporel, par exemple du sang. Il peut notamment s'agir de sang total. Les particules 21 peuvent être des particules sanguines, et plus particulièrement des globules rouges. Il peut également s'agir de cellules, de microorganismes, par exemple des bactéries ou des levures, des microalgues, des microbilles, ou des gouttelettes insolubles dans le milieu liquide, par exemple des nanoparticules lipidiques. De préférence, les particules 21 ont un diamètre, ou sont inscrites dans un diamètre, inférieur à 1 mm, et de préférence inférieur à 100 $\mu$m. Il s'agit de microparticules (diamètre inférieur à 1 mm) ou de nanoparticules (diamètre inférieur à 1 $\mu$m). Le milieu liquide 22, dans lequel baignent les particules, peut être une phase liquide d'un liquide corporel, d'un milieu de culture ou d'un liquide prélevé dans l'environnement ou dans un procédé industriel.

La distance D entre la source de lumière 11 et l'échantillon 20 est de préférence supérieure à 1 cm. Elle est de préférence comprise entre 1 et 30 cm, typiquement 5 cm.

La source de lumière 11 peut être une diode électroluminescente ou une source de lumière laser comme une diode laser. La source de lumière 11 peut comporter un filtre optique 19, notamment un filtre passe-bande, permettant d'ajuster la bande spectrale d'illumination de l'onde lumineuse incidente 12. Un tel filtre optique est optionnel. La bande spectrale d'illumination de l'onde lumineuse émise par la source de lumière 11 est adaptée à un spectre d'absorption d'un indicateur coloré 24 décrit dans la suite de la description.

L'échantillon 20 est contenu dans une chambre fluidique 15. La chambre fluidique 15 est par exemple une micro-cuvette, d'utilisation courante dans les dispositifs de type point of care, dans laquelle l'échantillon 20 pénètre, par exemple par capillarité. Sur la figure 1A, on a représenté deux parois longitudinales 16, 17, désignées respectivement par les termes paroi supérieure et paroi inférieure, transparentes et distantes de 150 $\mu$m. La distance entre ces deux parois longitudinales 16, 17, selon l'axe de propagation Z, correspond à l'épaisseur e de l'échantillon. Cette dernière varie typiquement entre 20 $\mu$m et 1 cm, et est de préférence comprise entre 50 $\mu$m et 500 $\mu$m, par exemple 150 $\mu$m. Les parois latérales de la chambre fluidique, s'étendant parallèlement à l'axe de propagation Z, ne sont pas représentées.

La chambre fluidique 15 est disposée entre la source de lumière 11 et un capteur d'image 30, apte à établir une image Im, dite image de transmission, d'une onde lumineuse 14 transmise par l'échantillon 20. Le capteur d'image 30 s'étend selon un plan de détection P, de préférence parallèlement, ou sensiblement parallèlement aux parois longitudinales 16, 17 de la chambre fluidique 15. Le terme sensiblement parallèlement signifie que les deux éléments peuvent ne pas être rigoureusement parallèles, une tolérance angulaire de quelques degrés, inférieure à 20° ou à 10° étant admise.

Le capteur d'image 30 comporte une matrice de pixels, de type CCD (de l'anglais Charge Coupled Device) ou un CMOS (de l'anglais Complementary Metal-Oxyde Semiconductor). Les capteurs d'image dont le pas inter pixel est inférieur à 3 $\mu$m sont préférés, car ils permettent d'obtenir des images avec une résolution spatiale satisfaisante.

On remarque, dans cet exemple, l'absence d'optique de grossissement entre le capteur d'image 30 et l'échantillon 20. Cela n'empêche pas la présence éventuelle de microlentilles de focalisation au niveau de chaque pixel du capteur d'image 30. Cela permet de former une image Im, dite de transmission, d'une onde lumineuse 14 transmise par l'échantillon en minimisant la distance entre l'échantillon 20 et le capteur d'image 30. Cela permet d'utiliser un dispositif d'analyse particulièrement simple et compact. Ainsi, en l'absence d'optique de grossissement, la distance d entre l'échantillon et les pixels du photodétecteur est de préférence inférieure à 2 cm, voire à 1 cm, et préférentiellement comprise entre 50 $\mu$m et 2 cm, de préférence comprise entre 100 $\mu$m et 2 mm.

Un processeur 32, par exemple un microprocesseur, est apte à traiter les images Im acquises par le capteur d'image 30. En particulier, le processeur est un microprocesseur relié à une mémoire programmable 33 dans laquelle est stockée une séquence d'instructions pour effectuer les opérations de traitement d'image et de calcul décrites dans cette description. Le processeur peut être relié à un écran d'affichage 34.

La chambre fluidique 15 est reliée mécaniquement à un premier transducteur électromécanique 41, apte à propager une onde acoustique ultrasonore dans l'échantillon 20. Le terme ultrasonore désigne le fait que la fréquence de l'onde acoustique est supérieure à 15 kHz et inférieure à 1 GHz. Dans cet exemple, le premier transducteur 41 est un transducteur piézoélectrique, apte à entraîner une vibration d'une paroi de la chambre fluidique 15, en l'occurrence la paroi supérieure 16, ce qui entraîne la formation d'une onde acoustique 45 se propageant dans l'échantillon 20, et cela à une fréquence d'excitation du transducteur piézoélectrique. La fréquence d'excitation correspond généralement à une fréquence de résonance du transducteur 41, le fonctionnement de ce dernier étant optimal à une telle fréquence. Cette fréquence de résonance dépend du transducteur, ce dernier pouvant également être désigné par le terme résonateur. Généralement, un transducteur piézoélectrique comporte un matériau piézoélectrique disposé entre deux électrodes. Sa fréquence de résonance dépend de sa forme, notamment son épaisseur. Le premier transducteur 41 peut être directement placé au contact de la chambre fluidique 15, ou placé sur un élément d'interface, ce dernier assurant un meilleur couplage entre l'onde mécanique produite par le transducteur et l'onde acoustique formée dans l'échantillon 20.

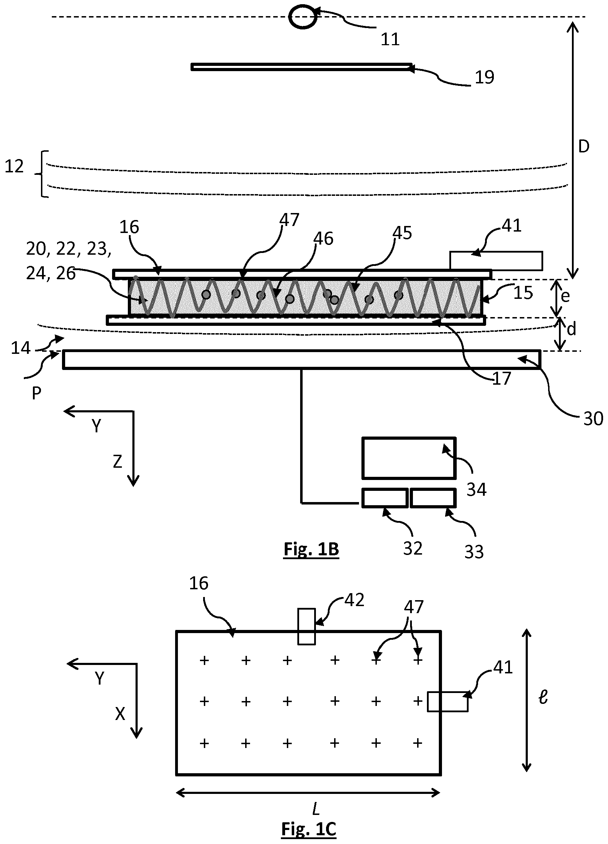

Les particules 21 en suspension dans l'échantillon 20 se trouvent alors placées dans un champ de pression acoustique, et subissent une force, dite force acoustique, pouvant engendrer un déplacement desdites particules. En fonction d'un paramètre caractérisant ces particules, désigné par le terme facteur de contraste, les particules se concentrent soit dans les nœuds de pression 46, c'est-à-dire les zones de l'échantillon dans lesquelles l'amplitude de l'onde de pression est minimale, comme représenté sur la figure 1B, ou dans les ventres de pression 47, c'est-à-dire les zones de l'échantillon dans lesquelles l'amplitude de l'onde de pression est maximale. Ainsi, le recours à une onde acoustique 45 permet d'obtenir une concentration des particules 21 selon le champ de pression formé dans l'échantillon et du facteur de contraste des particules. Lorsque l'échantillon 20 comporte des particules sanguines, il a été observé que les globules rouges ont tendance à s'accumuler au niveau des nœuds de pression 46. Le recours à une onde acoustique 45 permet alors une segmentation spatiale de l'échantillon 20 en des zones 20a appauvries en particules, ou zones déplétées, et en des zones 20b enrichies en particules. L'invention permet une séparation des particules sans déplacement de la chambre fluidique 15 dans laquelle l'échantillon est confiné. Dans l'échantillon, les particules 21 ne subissent pas un mouvement de rotation autour d'un même axe de rotation, comme dans les procédés de centrifugation de l'art antérieur.

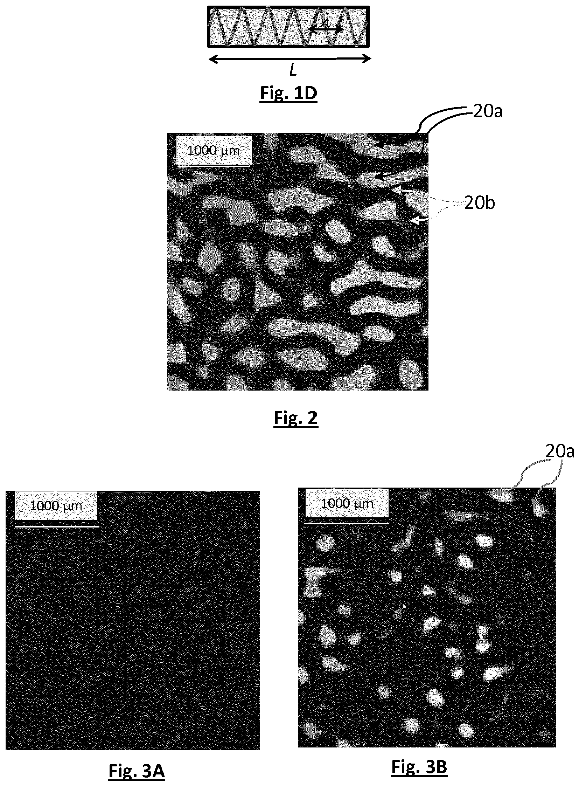

De préférence, l'onde acoustique 45 appliquée à l'échantillon 20 est stationnaire. Dans ce cas, la longueur d'onde λ de l'onde acoustique 45 est avantageusement déterminée en fonction des dimensions de la chambre fluidique 15. Une condition peut notamment être qu'une dimension longitudinale L, selon laquelle se propage l'onde acoustique, est un multiple de la demi-longueur d'onde. Cette condition, illustrée sur la figure 1D, est notamment utile lorsque ladite dimension longitudinale est faible, typiquement de l'ordre de la longueur d'onde λ ou de quelques longueurs d'onde. La formation d'une onde stationnaire est avantageuse, car elle permet d'obtenir des ventres ou des nœuds de pression dont la position n'évolue pas significativement dans le temps. La dimension longitudinale L de la chambre fluidique peut être supérieure à 10 fois la longueur d'onde λ, auquel cas il est possible de former une onde pseudo-stationnaire sans ajustement précis de la longueur d'onde vis-à-vis de cette dimension. Le terme pseudo-stationnaire désigne le fait que l'onde comporte une composante progressive, mais que le déplacement des ventres 47 ou des nœuds de pression 46 est suffisamment faible pour que le déplacement des particules 21, sous l'effet de cette composante progressive, soit négligeable pendant la durée d'acquisition, par le capteur d'image 30, d'une image Im de l'onde lumineuse 14 transmise par l'échantillon 20. La durée d'une telle acquisition est typiquement de l'ordre de 1 seconde, ou est inférieure ou égale à 1 seconde.

La figure 1C représente un mode de réalisation selon lequel le chambre fluidique est couplée à deux transducteurs 41 et 42, orientés orthogonalement l'un à l'autre. Un premier transducteur 41 est apte à former une première onde acoustique se déplaçant selon l'axe Y, tandis qu'un deuxième transducteur 42 est apte à former une deuxième onde acoustique se déplaçant selon l'axe X. Cette configuration permet une répartition bidimensionnelle des nœuds et des ventres de pression dans le plan XY. On a schématisé, sur la figure 1C, une répartition matricielle des ventres de pression 47 dans le cas idéal dans lequel le premier transducteur 41 et le deuxième transducteur 42 forment respectivement une onde stationnaire de même longueur d'onde, ce qui conduit à une répartition régulière et bidimensionnelle des ventres de pression 47.

La figure 2 représente une image expérimentale d'un premier échantillon de volume 80 μl comportant 46.8 μl de sang dilué dans 33.2 μl de tampon Tris-Glycine. Pour obtenir cette image, l'échantillon 20 a été disposé dans une chambre fluidique 15 de forme parallélépipédique, de longueur L = 2 cm, de largeur ℓ = 2 cm et d'épaisseur e = 150 μm. Cette chambre fluidique est placée à 5 cm d'une source de lumière. La source de lumière est une diode électroluminescente émettant dans une bande spectrale d'émission centrée sur une longueur d'onde de 660 nm. Il s'agit d'une diode électroluminescente fournie par le fournisseur CoolLED, sous la référence pE illumination system 244 5100 red. Il est préférable que la largeur de la bande spectrale d'émission soit inférieure à 100 nm, cette largeur correspondant à une largeur à mi-hauteur du pic d'émission.

La chambre fluidique 15 comporte une lame supérieure transparente 16, en verre, et d'épaisseur 150 μm. Elle comporte également une lame inférieure 17, en verre, d'épaisseur 1 mm. La lame inférieure est disposée à une distance d de 1 cm du capteur d'image 30, ce dernier étant un capteur CMOS Hamamatsu digital camera C11440 22 CU.

La plaque supérieure 16 est reliée à deux transducteurs piézoélectriques Noliac NCE 51 orientés orthogonalement l'un à l'autre, comme représenté sur la figure 1C. Chaque transducteur est alimenté par un signal d'excitation dont la tension crête-crête est de 14 V, un déphasage de 90° étant appliqué entre le signal d'excitation du premier transducteur 41 et le signal d'excitation du deuxième transducteur 42. La fréquence de résonance de ces transducteurs est de 3 MHz. On a tout d'abord ajusté expérimentalement une fréquence de résonance de la chambre fluidique 15, tenant compte du couplage entre les transducteurs 41, 42 et ladite chambre fluidique, ainsi que de l'impédance acoustique de l'échantillon 20. Cette détermination a été effectuée en utilisant un échantillon de calibration constitué d'une solution aqueuse comportant des billes de polystyrène de diamètre 40 μm. On a observé, visuellement, qu'une segmentation optimale des billes de polystyrène se produisait avec une fréquence du signal d'excitation de 2.415 MHz.

Une telle fréquence d'excitation a été appliquée sur les transducteurs 41, 42 après que la chambre fluidique 15 a été remplie avec l'échantillon de calibration précédemment décrit. La figure 2 représente une image obtenue après activation des transducteurs. On observe des régions claires 20a réparties selon une distribution bidimensionnelle, ainsi que des régions sombres 20b. Les régions sombres correspondent aux nœuds de pression, dans lesquelles les particules se concentrent, pour former les parties enrichies 20b. L'onde lumineuse incidente 12 subie une importante atténuation par ces particules, ce qui explique la faible intensité de l'onde 14 transmise par l'échantillon 20 au niveau de ces parties 20b concentrées en particules. A l'inverse, les régions claires correspondent aux parties 20a de l'échantillon appauvries en particules 21 du fait de l'application de l'onde acoustique 45. Cette image montre que sous l'effet de l'onde acoustique 45, la transmission optique de l'échantillon n'est plus homogène : elle est faible dans les parties 20b de l'échantillon enrichies en particules et élevée dans les parties appauvries 20a.

Par transmission optique, on entend une comparaison entre l'intensité $i_{14}$ de l'onde transmise par l'échantillon sur l'intensité $i_{12}$ de l'onde incidente à l'échantillon. La transmission optique $Tr$ est classiquement exprimée sous la forme d'une comparaison, comparaison pouvant notamment prendre la forme d'un ratio

$$Tr = \frac{i_{14}}{i_{12}}$$

tel que $Tr$ Sans application de l'onde acoustique 45, la transmission optique d'un échantillon est homogène, du fait de la répartition homogène des particules. L'application de l'onde acoustique 45 permet d'obtenir une distribution spatiale hétérogène de la transmission optique, cette dernière présentant des minimas au niveau des parties enrichies 20b et des maximas au niveau des parties appauvries 20a. Lorsque l'échantillon est du sang, chaque partie appauvrie est essentiellement composée de plasma et chaque partie enrichie est essentiellement composée de globules rouges. L'application de l'onde acoustique, couplée à une détection d'une onde lumineuse transmise par l'échantillon, au niveau d'une partie appauvrie 20a, permet alors d'estimer la transmission optique $Tr$ du plasma.

D'une façon plus générale, et c'est un point essentiel de l'invention, la combinaison d'une onde acoustique et de la mesure de l'intensité de l'onde lumineuse transmise par un échantillon permet d'estimer une transmission optique d'une zone appauvrie en particules de l'échantillon.

Les inventeurs ont mis en œuvre l'invention pour effectuer un dosage d'un analyte 26, et en particulier du glucose, dans le sang. Les principes de détection du glucose dans un échantillon sanguin par la mise en œuvre de réactions enzymatiques aboutissant à la formation d'un indicateur coloré sont décrits dans les brevets US3964974 et US5866349. D'une façon générale, cette méthode colorimétrique est basée sur :

- l'oxydation du glucose par NAD (acronyme de nicotinamide adenine dinucleotide) agissant en tant que cofacteur, en présence de GDH (acronyme de glucose dehydrogenase), aboutissant à la formation de NADH + H$^+$ (acronyme de acide dihydronicotinique amide adenine dinucleotide) ;
- la réduction d'un sel de tétrazolium par NADH + H$^+$, en présence de diaphorase (dihydrolipoyl dehydrogenase), réaction aboutissant à la formation d'un indicateur coloré 24, dont la concentration est représentative de la concentration de glucose dans l'échantillon.

Le sel de tétrazolium utilisé peut être du MTT, acronyme de bromure de 3-(4,5-diméthylthiazolyl-2-yl)-2,5-diphényltetrazolium, auquel cas l'indicateur coloré est le Formazan, de couleur violette.

Le terme indicateur coloré désigne une espèce chimique ayant une couleur particulière, et dont la formation dans l'échantillon est apte à modifier le spectre d'absorption ou le spectre de transmission de l'échantillon.

Aussi, le procédé comporte une étape de mélange de l'échantillon 20 avec un réactif 23, permettant la formation d'un indicateur coloré 24 en réagissant avec l'analyte 26 présent dans l'échantillon 20, cet analyte étant, dans cet exemple, le glucose. Le réactif 23 peut comporter de la GDH, du NAD, de la Diaphorase et du MTT.

La formation de l'indicateur coloré 24 représentatif de l'analyte 26 entraîne une diminution de la transmission optique de l'échantillon, et notamment dans le plasma, dans une bande spectrale d'absorption (ou bande spectrale de coloration) de l'indicateur coloré. Il est connu que cette bande spectrale s'étend entre 370 et 700 nm, avec un maximum d'absorption vers λ=565 nm. Ainsi, lorsqu'un dosage de glucose est mis en œuvre, il est usuel de déterminer l'atténuation optique de l'échantillon analysé à 660 nm, cette dernière étant d'autant plus marquée que la concentration de glucose est importante. L'atténuation *Att* désigne le complémentaire de la transmission, de telle sorte que *Att* = 1 - *Tr*.

Un échantillon de référence a été constitué. Cet échantillon de référence comporte 46.8 μl de sang, la concentration en glucose étant de 20 mM, 20 μl de MTT et 13.2 μl de tampon salin. L'échantillon de référence ne comporte ni le cofacteur (NAD) ni les enzymes, en l'occurrence GDH, permettant, sous l'effet du glucose, la formation de l'agent (NADH + H$^+$) responsable de la réduction du MTT en un indicateur coloré. La figure 3A représente une image de l'échantillon 20 de référence avant activation des transducteurs 41,42. La figure 3B montre une image de l'échantillon de référence après application d'une onde acoustique, dans l'échantillon 20, par les transducteurs. A l'instar de la figure 2, on observe la formation de régions appauvries claires 20a et des régions enrichies sombres 20b.

On a ensuite réalisé des images de d'un premier et d'un deuxième échantillon de test, ces échantillons étant désignés par *test$_1$* et *test$_2$*. Chaque échantillon de test a la composition suivante :

- sang : 46.8 μl ;
- MTT : 20 μl ;
- Tampon salin : 10 μl ;
- Mutarotase : 0.8 μl ;
- GDH : 0.8 μl ;
- Diaphorase : 0.8 μl ;
- NAD : 0.8 μl.

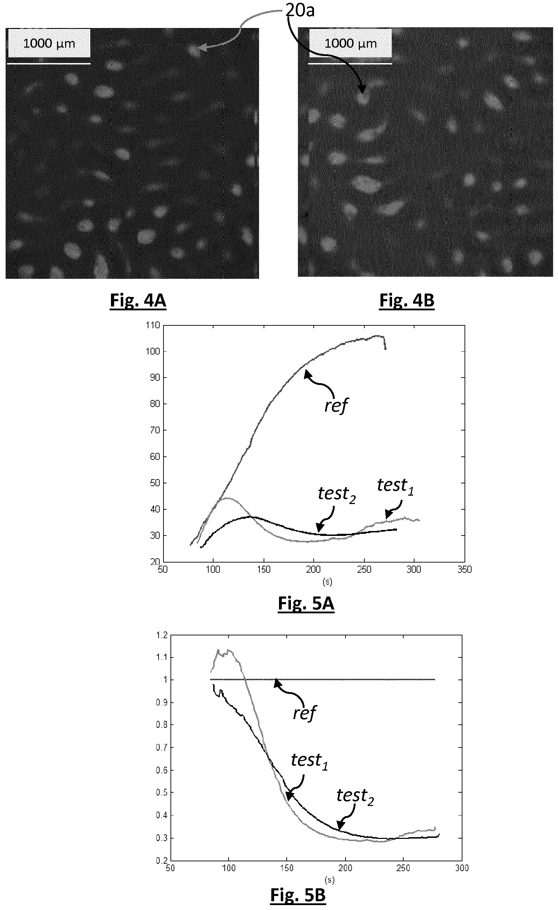

Les figures 4A et 4B représentent des images respectives du premier et du deuxième échantillon de test, ces images ayant été acquises respectivement 172 secondes et 175 secondes après ajout du réactif 23 permettant la formation de l'indicateur coloré 24. On observe une séparation du plasma et des particules sanguines, essentiellement les globules rouges, les zones claires 20a permettant de déterminer la transmission optique du plasma. Ces images ont été répétées dans le temps, à une cadence de 1 image par seconde. On a observé un assombrissement progressif des zones claires 20a, sous l'effet de la formation de l'indicateur coloré 24 du fait des réactions enzymatiques préalablement décrites.

Les inventeurs ont quantifié l'évolution de l'intensité $i_{14}$ de l'onde 14 transmise par l'échantillon de référence *ref* et les deux échantillons de test *test$_1$*, *test$_2$*, en limitant l'analyse des images acquises aux régions d'intérêts formées par les différentes parties 20a appauvries en particules. Pour cela, un seuillage en intensité a été appliqué sur chacune de ces images, de façon à exclure les différentes régions sombres de l'analyse, ces dernières étant représentatives des parties enrichies 20b de l'échantillon 20. De ce fait, chaque image seuillée ne comporte que les régions claires, correspondant aux parties appauvries 20a. Sur chaque image seuillée, l'intensité moyenne des pixels des différentes zones claires a été déterminée. La figure 5A représente l'évolution, en fonction du temps, de l'intensité moyenne des pixels des zones claires, et cela respectivement pour l'échantillon de référence *ref*, le premier échantillon de test *test$_1$* et le deuxième échantillon de test *test$_2$*. L'unité de l'axe des abscisses est la seconde, tandis que l'axe des ordonnées représente l'intensité moyenne exprimée en niveaux de gris. Pour chaque échantillon de test, l'instant initial, à l'origine de l'axe des abscisses, correspond à l'ajout du réactif 23 permettant le déclenchement de la réaction enzymatique aboutissant à la formation de l'indicateur coloré 24. Les courbes sont tracées à partir de l'instant correspondant à l'actionnement des transducteurs.

Sur l'échantillon de référence *ref*, l'intensité moyenne des zones claires augmente puis tend à se stabiliser, témoignant d'une segmentation progressive de l'échantillon en parties claires 20a et en parties sombres 20b.

Sur les deux échantillons de test, l'intensité moyenne augmente, sous l'effet de la segmentation, puis diminue et se stabilise à des niveaux comparables, du fait de la formation progressive de l'indicateur coloré, tendant à assombrir lesdites parties appauvries 20a.

Sur l'échantillon de référence et les deux échantillons de test, les inventeurs ont normalisé l'intensité de chaque image seuillée par l'intensité moyenne, au même instant, de l'image seuillée de l'échantillon de référence. La courbe représenté sur la figure 5B représente, pour chaque échantillon, l'évolution, en fonction du temps, de l'intensité de chaque image seuillée sur l'intensité moyenne de l'image seuillée de l'échantillon de référence. En considérant que l'intensité moyenne des zones claires de l'image de référence correspond à l'intensité de l'onde incidente, on obtient alors une grandeur représentative la transmission optique du plasma, c'est-à-dire du milieu liquide 22 dans lequel baignent les globules rouges, en fonction du temps. On constate une diminution significative de cette transmission, puis une stabilisation. Ainsi, en se basant sur un étalonnage, réalisée avec des échantillons de test dans lesquels la quantité d'analyte est connue, l'invention permet une estimation d'une quantité d'analyte dans un échantillon inconnu, sur la base de l'intensité moyenne des différentes zones claires de l'image.

L'étape d'ajout d'un réactif 23 apte à modifier une propriété optique de l'échantillon en fonction d'une quantité d'analyte 26 est optionnelle. L'invention peut également être appliquée à l'analyse d'une propriété optique d'une ou plusieurs zones appauvries 20a de l'échantillon, de façon à caractériser le milieu liquide 22 dans lequel baignent les particules 21. Lorsque l'échantillon est du sang, l'invention permet d'observer, par exemple, une coloration pathologique du plasma. Dans ce cas, les forces acoustiques permettent une segmentation de l'échantillon en parties appauvries 20a et en parties enrichies 20b, comme préalablement décrit. Le capteur d'image 30 permet de visualiser une image *Im* représentative de cette segmentation. A partir de chaque zone claire de l'image, correspondant à une partie appauvrie 20a, une estimation d'une propriété optique du milieu liquide 22 dans lequel baignent les particules 21, est réalisée. Il peut s'agir d'une détermination de la couleur ou d'une détermination d'une transmission optique ou d'une absorption optique du milieu liquide 22. Ce procédé peut comprendre une étape de comparaison de ladite propriété optique estimée avec une propriété optique d'un échantillon de référence connu, déterminée lors d'un étalonnage.

Une application de l'invention peut concerner le domaine de la production de microalgues. L'invention permet de suivre l'évolution du milieu liquide dans lequel baignent les microalgues, par exemple en examinant un changement de couleur sous l'effet du relargage de chlorophylle par lesdites microalgues. L'invention peut être également être appliquée à la culture cellulaire, en examinant une éventuelle évolution d'une propriété optique d'un milieu de culture, par exemple sous l'effet d'une variation du pH. Le milieu de culture peut alors comporter un indicateur coloré dont la couleur change en fonction du pH.

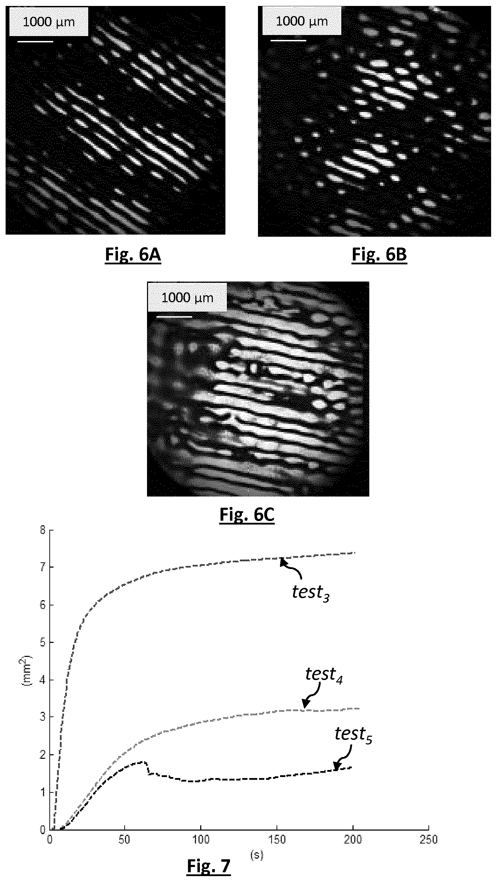

Les figures 6A à 6C représentent des images acquises en observant du sang dans lequel le taux d'hématocrite est variable. Plus le taux d'hématocrite est élevé, plus l'aire des parties appauvries 20a diminue et plus l'aire des parties enrichies 20b augmente. Sur les images acquises par le capteur d'image 30, plus le taux d'hématocrite est élevé, plus la surface des zones claires diminue, et plus la surface des zones sombres augmente.

Les figures 6A à 6C ont été obtenues en utilisant trois échantillons de test *test$_3$, test$_4$, test$_5$* obtenus à partir de sang humain, dans lesquels le taux d'hématrocrite est respectivement égal à 41%, 25% et 10%. Ces échantillons ont été obtenus par prélèvement sanguin sur donneur humain, le sang prélevé présentant un taux d'hématocrite de 41%. Les taux d'hématrocrite de 10% et 25% résultent d'une dilution du sang par son propre plasma, ce dernier ayant été extrait par centrifugation. Les figures 6A à 6C ont été acquises en mettant en œuvre un dispositif similaire à celui décrit en lien avec la détermination de la glycémie, un seul transducteur piézoélectrique étant utilisé. Le volume de chaque échantillon de test est de 90 $\mu$l.

On a acquis des images *Im* à l'aide du capteur d'images 30 précédemment décrit. On a sélectionné, sur chaque image *Im* acquise par le capteur d'image, une région d'intérêt dans laquelle la segmentation de l'image en zones sombres et zones claires est stable dans le temps.

Les figures 6A à 6C représentent cette région d'intérêt respectivement pour les trois échantillons considérés. La figure 7 montre, dans chaque région d'intérêt, l'évolution temporelle de la surface cumulée des zones claires, cette dernière étant exprimée en mm$^2$. Cette sur-face cumulée est représentative des zones appauvries 20a dans chaque échantillon de test. De même que pour l'exemple précédent, cette surface cumulée a été obtenue par un seuillage en intensité de façon à exclure les différentes zones sombres dont le niveau d'intensité est inférieur à un seuil. On observe également qu'au bout d'une durée d'environ 65 secondes, l'aire de la surface cumulée se stabilise, ce qui témoigne d'une certaine stabilité dans la segmentation des particules.

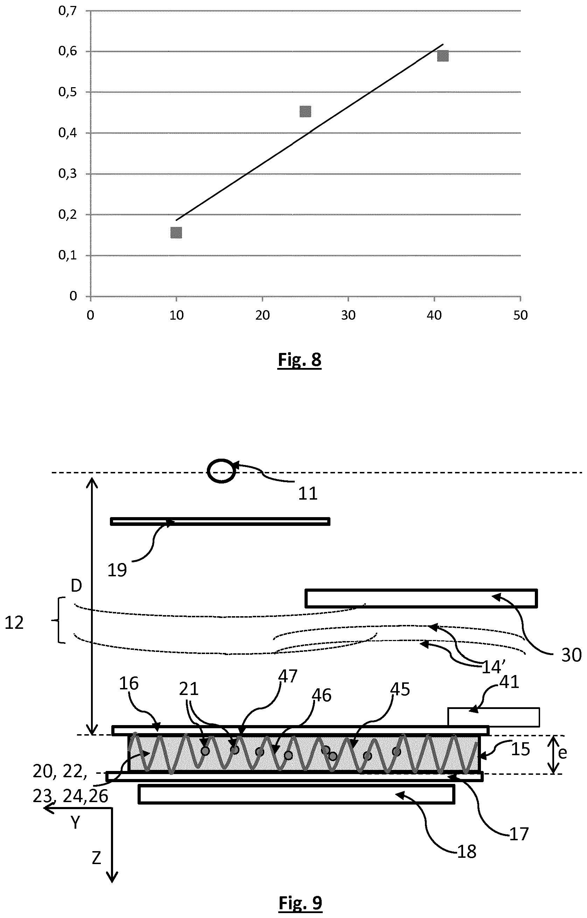

On observe que plus le taux d'hématocrite est élevé, plus la surface cumulée des zones claires de l'image diminue. Ainsi, sur la base d'un étalonnage à l'aide d'échantillons de test connus, hors de l'invention revendiquée, il est possible de déterminer un taux d'hématocrite par une segmentation de l'échantillon 20 en zones appauvries 20a et zones enrichies 20b, et par une observation de la surface représentative de ces zones, ou d'un ratio entre lesdites zones. La figure 8 montre une courbe d'étalonnage, obtenue sur la base des échantillons *test$_3$, test$_4$, test$_5$,* et permettant d'estimer un taux d'hématocrite (axe des abscisses, l'unité étant le %) en fonction de l'inverse la surface cumulée des zones appauvries 20a dans un échantillon (axe des ordonnées, l'unité étant le mm$^{-2}$). De façon plus générale, hors de l'invention revendiquée, il est possible d'estimer une quantité de particules 21 présentes dans l'échantillon liquide 20.

Hors de l'invention revendiquée, on peut réaliser des mesures de fluorescence. L'onde lumineuse incidente 12 peut induire une fluorescence de l'échantillon 20

Ainsi, l'invention permet de caractériser un échantillon 20 comportant des particules 21 baignant dans un milieu liquide 22,

- en déterminant une propriété optique, ou son évolution au cours du temps, du milieu liquide 22 dans lequel baignent les particules 21. Lorsque cette propriété optique varie en fonction d'un analyte, l'invention permet de déterminer la quantité dudit analyte dans l'échantillon.

Dans les essais qui précèdent, on a mis en œuvre un capteur d'image 30, ce qui constitue une configuration avantageuse. En effet, à l'aide d'un tel capteur, les parties appauvries et enrichies peuvent être aisément identifiées et segmentées par des procédés simples de traitement d'image. De plus, l'onde acoustique 45 n'est pas forcément stationnaire et l'invention est applicable, à l'aide d'un capteur d'image, lorsque la variation spatiale des zones appauvries 20a et des zones enrichies 20b est négligeable devant la durée d'acquisition d'une image. Cependant, l'utilisation d'un photodétecteur non résolu spatialement, par exemple une photodiode, est possible, mais cela suppose une connaissance préalable de la localisation des zones enrichies 20b ou des zones appauvries 20a, de telle sorte le photodétecteur soit :

- soit couplé optiquement à une zone appauvrie ou à une zone enrichie ;
- soit disposé en regard d'une zone appauvrie ou d'une zone enrichie.

Cela suppose la formation d'une onde acoustique stationnaire et une localisation précise du photodétecteur par rapport auxdites zones appauvries 20a ou desdites zones enrichies 20b. Le recours à un capteur d'image est moins contraignant, les zones appauvries et enrichies pouvant être identifiées sur les images acquises par le capteur selon des procédés classiques de segmentation, tels le seuillage.

Selon un mode de réalisation, représenté sur la figure 9, le capteur d'image 30 est placé selon une géométrie de réflexion, et non en transmission comme dans les modes de réalisation précédemment décrits. Le capteur d'image 30 est apte à former une image d'une onde 14' transmise par l'échantillon, après avoir été réfléchie par un élément réfléchissant 18. Ce dernier peut être un support réfléchissant sur lequel est disposée la chambre fluidique 15, soit un dépôt réfléchissant formé sur la lame inférieure 17. L'élément réfléchissant 18 peut être une surface blanche, une surface métallisée ou un miroir. Le dispositif 1' illustré sur la figure 9 est un dispositif en réflexion.

Dans les exemples décrits ci avant, l'échantillon est confiné dans une chambre fluidique 15. L'invention s'applique également à un échantillon déposé sur un support fluidique, ce dernier pouvant se limiter à une simple lame transparente, l'échantillon étant déposé sur cette lame.

Au-delà du dosage du glucose dans un échantillon sanguin, l'invention pourra s'appliquer au dosage d'un analyte dans un liquide comportant des particules, en mettant en œuvre une segmentation avantageuse du liquide en au moins une zone appauvrie en particules et une zone enrichie en particules. L'échantillon peut notamment comprendre un liquide corporel, l'analyte pouvant être le glucose, le cholesterol, ou d'autres éléments tels des protéines.

L'invention trouvera des applications dans la caractérisation de liquides corporels, à des fins d'aide à un diagnostic. Mais les applications dépassent le domaine des liquides corporels et l'invention pourra être mise en œuvre pour caractériser des échantillons liquides prélevés dans l'environnement, ou concernant différents domaines industriels, par exemple, et de façon non exhaustive le domaine de la culture cellulaire, le domaine de l'agroalimentaire, le domaine de la culture de microorganismes tels que des microalgues.

**Revendications**

1. Procédé de caractérisation d'un échantillon (20), comportant des particules (21) baignant dans un milieu liquide (22), le procédé comprenant les étapes suivantes :

a) illumination de l'échantillon (20) à l'aide d'une source de lumière (11), apte à émettre une onde lumineuse incidente (12) se propageant vers l'échantillon ;
b) l'acquisition, à l'aide d'un capteur d'image (30), d'au moins une image de l'onde lumineuse (14, 14') transmise par l'échantillon ainsi illuminé ;
c) caractérisation de l'échantillon en fonction d'une intensité de l'onde lumineuse détectée par le capteur d'image ;

le procédé comprenant, préalablement à l'étape b), une étape d'application d'une onde acoustique (45) dans l'échantillon, ladite onde acoustique (45) formant des nœuds de pression (46) et des ventres de pression (47) dans l'échantillon, de manière à séparer, dans ce dernier, une partie appauvrie en particules (20a) et une partie enrichie en particules (20b), le procédé étant **caractérisé en ce que** l'étape c) comporte :

- une identification, sur l'image, d'une région d'intérêt correspondant à la partie (20a) de l'échantillon, appauvrie en particules ;
- une détermination d'une propriété optique du milieu liquide, dans la région d'intérêt (20a) ainsi identifiée, la propriété optique variant en fonction d'une quantité d'un analyte (26) dans l'échantillon ;
- une estimation de la quantité de l'analyte (26), dans le milieu liquide (22), à partir de ladite propriété optique, dans la région d'intérêt (20a).

2. Procédé selon la revendication 1, dans lequel les particules (21) présentes dans l'échantillon se concentrent, sous l'effet de ladite onde acoustique (45), soit au niveau des nœuds de pression (46), soit au niveau des ventres de pression (47).

3. Procédé selon l'une quelconque des revendications précédentes, comportant, préalablement à l'étape b), un mélange de l'échantillon (20) avec un réactif (23) apte à modifier la propriété optique du milieu liquide (22) sous l'effet de l'analyte (26).

4. Procédé selon la revendication 3, dans lequel le réactif (23) est apte à former un indicateur coloré (24) dans le milieu liquide (22), sous l'effet de l'analyte (26).

5. Procédé selon la revendication 4, dans lequel l'échantillon (20) comporte des cellules dans un milieu de culture cellulaire, l'indicateur coloré étant tel que sa couleur change en fonction du pH du milieu de culture.

**6.** Procédé selon la revendication 4, dans lequel l'échantillon comporte des globules rouges dans du plasma sanguin, l'indicateur coloré étant tel que sa couleur change en fonction d'une quantité de glucose dans le plasma.

**7.** Procédé selon l'une quelconque des revendications précédentes, dans lequel l'échantillon est maintenu sur un support fluidique (15), l'application de l'onde acoustique (45) étant réalisée au moyen d'au moins un transducteur électromécanique (41, 42), notamment piézoélectrique, agissant sur ledit support fluidique de façon à propager l'onde acoustique (45) dans ledit échantillon.

**8.** Procédé selon l'une quelconque des revendications précédentes, dans lequel l'onde acoustique (45) appliquée à l'échantillon (30) est une onde stationnaire.

**9.** Procédé selon l'une quelconque des revendications 1 à 2, dans lequel l'échantillon comprend un liquide corporel et l'analyte est du glucose ou du cholesterol.

**10.** Procédé selon l'une quelconque des revendications 1 à 2, dans lequel l'échantillon comprend un liquide corporel et l'analyte est une protéine.

**11.** Dispositif (1, 1') pour caractériser un échantillon (20), comportant des particules (21) baignant dans un milieu liquide (22), le dispositif comportant :

   - un support fluidique (15), apte à maintenir l'échantillon ;
   - une source de lumière (11), agencée pour émettre une onde lumineuse incidente (12) se propageant vers le support fluidique (15), apte à maintenir l'échantillon (20);
   - un capteur d'image (30), apte à acquérir une image de l'onde lumineuse (14, 14') transmise par l'échantillon (20), maintenu sur le support fluidique (15), lorsqu'il est illuminé par ladite onde lumineuse incidente ;
   - un processeur (32), apte à caractériser l'échantillon (20) en fonction d'une intensité de l'onde lumineuse détectée par le capteur d'image (30) ; et un transducteur électromécanique (41,42), configuré pour appliquer une onde acoustique (45) se propageant dans l'échantillon (20), maintenu sur le support fluidique (15), de manière à séparer, dans l'échantillon (20), une partie appauvrie (20a) en particules et une partie enrichie (20b) en particules, le dispositif étant **caractérisé en ce que** le processeur (32) est configuré pour:
   - identifier, sur l'image, une région d'intérêt correspondant à au moins une partie (20a) appauvrie en particules ;
   - déterminer une propriété optique du milieu liquide, dans la région d'intérêt (20a) ainsi identifiée, la propriété optique variant en fonction d'une quantité d'un analyte (26) dans l'échantillon ; et
   - estimer la quantité de l'analyte (26) dans le milieu liquide (22), à partir de ladite propriété optique, dans la région d'intérêt.

**12.** Dispositif selon la revendication 11, dans lequel le transducteur électromécanique (41,42) comporte un transducteur piézoélectrique ou une pluralité de transducteurs piézoélectriques ;

**13.** Dispositif selon l'une quelconque des revendications 11 ou 12, dans lequel le transducteur électromécanique applique une pression sur ledit support fluidique (15), de manière à former une onde acoustique (45) dans l'échantillon (20) maintenu par ledit support fluidique.

**14.** Dispositif selon l'une quelconque des revendications 11 à 13, dans lequel il n'y a pas d'optique de grossissement entre le capteur d'image (30) et le support fluidique (15).

**Patentansprüche**

**1.** Verfahren zur Charakterisierung einer Probe (20), die Partikel (21) aufweist, welche in einem flüssigen Medium (22) schwimmen, wobei das Verfahren die folgenden Schritte umfasst:

   a) Beleuchten der Probe (20) mit Hilfe einer Lichtquelle (11), die dazu befähigt ist, eine einfallende Lichtwelle (12) auszusenden, die sich zur Probe hin ausbreitet;
   b) Aufnehmen, mit Hilfe eines Bildsensors (30), mindestens eines Bildes der Lichtwelle (14, 14'), wie sie durch die auf diese Weise beleuchtete Probe gelangt ist;
   c) Charakterisieren der Probe in Abhängigkeit von der Intensität der Lichtwelle, welche von dem Bildsensor detektiert wurde;

wobei das Verfahren, im Vorfeld des Schrittes b) einen Schritt des Einkoppelns einer Schallwelle (45) in die Probe umfasst, wobei die Schallwelle (45) Druckknoten (46) und Druckbäuche (47) in der Probe bildet, sodass sich in der letzteren ein Abschnitt, der an Partikeln abgereichert ist (20a), und ein Abschnitt, der an Partikeln angereichert ist (20b), voneinander trennen, wobei das Verfahren **dadurch gekennzeichnet ist, dass** der Schritt c) Folgendes aufweist:

   - ein Identifizieren, auf dem Bild, einer Region von Interesse, die dem Abschnitt (20a) der Probe entspricht, welcher an Partikeln abgereichert ist;

- ein Bestimmen einer optischen Eigenschaft des flüssigen Mediums in der auf diese Weise identifizierten Region von Interesse (20a), wobei die optische Eigenschaft in Abhängigkeit von einer Menge eines Analyten (26) in der Probe variiert;

- ein Abschätzen der Menge des Analyten (26) in dem flüssigen Medium (22), ausgehend von der optischen Eigenschaft, in der Region von Interesse (20a).

2. Verfahren nach Anspruch 1, wobei die Partikel (21), welche in der Probe vorliegen, sich unter Einwirkung der Schallwelle (45) entweder im Bereich der Druckknoten (46) oder im Bereich der Druckbäuche (47) konzentrieren.

3. Verfahren nach einem beliebigen der vorhergehenden Ansprüche, wobei es, im Vorfeld des Schrittes b), ein Vermischen der Probe (20) mit einem Reagenz (23) aufweist, welches dazu befähigt ist, die optische Eigenschaft des flüssigen Mediums (22) unter Einwirkung des Analyten (26) zu modifizieren.

4. Verfahren nach Anspruch 3, wobei das Reagenz (23) dazu befähigt ist, unter Einwirkung des Analyten (26) in dem flüssigen Medium (22) einen Farbindikator (24) zu bilden.

5. Verfahren nach Anspruch 4, wobei die Probe (20) Zellen in einem Zellkulturmedium aufweist, wobei der Farbindikator derart beschaffen ist, dass sich seine Farbe in Abhängigkeit von pH-Wert des Kulturmediums ändert.

6. Verfahren nach Anspruch 4, wobei die Probe rote Blutkörperchen in Blutplasma aufweist, wobei der Farbindikator derart beschaffen ist, dass sich seine Farbe in Abhängigkeit von der Menge an Glucose im Plasma ändert.

7. Verfahren nach einem beliebigen der vorhergehenden Ansprüche, wobei die Probe auf einem fluidartigen Träger (15) aufrechterhalten wird, wobei das Einkoppeln der Schallwelle (45) mittels mindestens eines elektromechanischen Wandlers (41, 42), insbesondere piezoelektrischer Art, erfolgt, wobei dieser derart auf den fluidartigen Träger einwirkt, dass sich die Schallwelle (45) in der Probe ausbreitet.

8. Verfahren nach einem beliebigen der vorhergehenden Ansprüche, wobei es sich bei der Schallwelle (45), welche in die Probe (30) eingekoppelt wird, um eine stehende Welle handelt.

9. Verfahren nach einem beliebigen der Ansprüche 1 bis 2, wobei die Probe eine Körperflüssigkeit umfasst und es sich bei dem Analyten um Glucose oder um Cholesterin handelt.

10. Verfahren nach einem beliebigen der Ansprüche 1 bis 2, wobei die Probe eine Körperflüssigkeit umfasst und es sich bei dem Analyten um ein Protein handelt.

11. Vorrichtung (1, 1') zur Charakterisierung einer Probe (20), die Partikel (21) aufweist, welche in einem flüssigen Medium (22) schwimmen, wobei die Vorrichtung Folgendes umfasst:

- einen fluidartigen Träger (15), der dazu befähigt ist, die Probe aufrechtzuerhalten;
- eine Lichtquelle (11), die derart angeordnet ist, dass sie eine einfallende Lichtwelle (12) aussendet, die sich zum fluidartigen Träger (15) hin ausbreitet, welcher dazu befähigt ist, die Probe (20) aufrechtzuerhalten;
- einen Bildsensor (30), der dazu befähigt ist, ein Bild der Lichtwelle (14, 14') aufzunehmen, wie sie durch die Probe (20) gelangt ist, welche auf dem fluidartigen Träger (15) aufrechterhalten wird, wenn sie von der einfallenden Lichtwelle beleuchtet wird;
- einen Prozessor (32), der dazu befähigt ist, die Probe (20) in Abhängigkeit von einer Intensität der Lichtwelle, welche von dem Bildsensor (30) detektiert wurde, zu charakterisieren; und

einen elektromechanischen Wandler (41, 42), der dafür ausgelegt ist, eine Schallwelle (45), die sich in der Probe (20) ausbreitet, welche auf dem fluidartigen Träger (15) aufrechterhalten wird, derart einzukoppeln, dass in sich in der Probe (20) ein Abschnitt, der an Partikeln abgereichert ist (20a), und ein Abschnitt, welcher an Partikeln angereichert ist (20b), voneinander trennen, wobei der Prozessor (32) dafür ausgelegt ist:

- auf dem Bild eine Region von Interesse zu identifizieren, die mindestens einem Abschnitt (20a) entspricht, der an Partikeln abgereichert ist;
- eine optische Eigenschaft des flüssigen Mediums in der auf diese Weise identifizierten Region von Interesse (20a) zu bestimmen, wobei die optische Eigenschaft in Abhängigkeit von einer Menge eines Analyten (26) in der Probe variiert; und
- die Menge des Analyten (26) in dem flüssigen Medium (22) abzuschätzen, ausgehend von der optischen Eigenschaft, in der Region von Interesse.

12. Vorrichtung nach Anspruch 11, wobei der elektromechanische Wandler (41, 42) einen piezoelektrischen Wandler oder eine Mehrzahl an piezoelektrischen Wandlern aufweist;

**13.** Vorrichtung nach einem beliebigen der Ansprüche 11 oder 12, wobei der elektromechanische Wandler einen Druck auf den fluidartigen Träger (15) ausübt, sodass sich in der Probe (20), welche von dem fluidartigen Träger aufrechterhalten wird, eine Schallwelle (45) bildet.

**14.** Vorrichtung nach einem beliebigen der Ansprüche 11 bis 13, wobei zwischen dem Bildsensor (30) und dem fluidartigen Träger (15) keinerlei vergrößernde Optik vorliegt.

**Claims**

**1.** Method for characterizing a sample (20), including a liquid medium (22) containing particles (21), the method comprising the following steps:

> a) illuminating the sample (20) using a light source (11) that is able to emit an incident light wave (12) that propagates towards the sample;
> b) acquiring, using an image sensor (30), at least one image of the light wave (14, 14') transmitted by the sample thus illuminated;
> c) characterizing the sample depending on an intensity of the light wave detected by the image sensor;

the method comprising, prior to step b), a step of applying an acoustic wave (45) to the sample, said acoustic wave (45) forming pressure nodes (46) and pressure antinodes (47) in the sample, so as to separate, in the latter, a portion (20a) poor in particles and a portion (20b) rich in particles, the method being **characterized in that** step c) includes:

> - identifying, in the image, a region of interest corresponding to the portion (20a) of the sample that is poor in particles;
> - determining an optical property of the liquid medium, in the region of interest (20a) thus identified, the optical property varying depending on an amount of an analyte (26) in the sample;
> - estimating the amount of the analyte (26) in the liquid medium (22), on the basis of said optical property in the region of interest (20a).

**2.** Method according to Claim 1, wherein the particles (21) present in the sample concentrate, under the effect of said acoustic wave (45), either level with the pressure nodes (46), or level with the pressure antinodes (47).

**3.** Method according to either one of the preceding claims, including, prior to step b), mixing the sample (20) with a reagent (23) able to modify the optical property of the liquid medium (22) under the effect of the analyte (26).

**4.** Method according to Claim 3, wherein the reagent (23) is able to form a coloured indicator (24) in the liquid medium (22), under the effect of the analyte (26).

**5.** Method according to Claim 4, wherein the sample (20) includes cells in a cellular culture medium, the coloured indicator being such that its colour changes depending on the pH of the culture medium.

**6.** Method according to Claim 4, wherein the sample includes red blood cells in blood plasma, the coloured indicator being such that its colour changes depending on an amount of glucose in the plasma.

**7.** Method according to any one of the preceding claims, wherein the sample is held by a fluid holder (15), the acoustic wave (45) being applied by means of at least one electromechanical and in particular piezoelectric transducer (41, 42) that acts on said fluid holder so as to propagate the acoustic wave (45) through the sample.

**8.** Method according to any one of the preceding claims, wherein the acoustic wave (45) applied to the sample (30) is a stationary wave.

**9.** Method according to either one of Claims 1 and 2, wherein the sample comprises a bodily liquid and the analyte is glucose or cholesterol.

**10.** Method according to either one of Claims 1 and 2, wherein the sample comprises a bodily liquid and the analyte is a protein.

**11.** Device (1, 1') for characterizing a sample (20), the sample including a liquid medium (22) containing particles (21), the device including:

> - a fluid holder (15) that is able to hold the sample;
> - a light source (11) that is arranged to emit an incident light wave (12) that propagates towards the fluid holder (15), which is able to hold the sample (20);
> - an image sensor (30) that is able to acquire an image of the light wave (14, 14') transmitted by the sample (20), which is held in the fluid holder (15), when it is illuminated by said incident light wave;
> - a processor (32) that is able to characterize the sample (20) depending on an intensity of the light wave detected by the image sensor (30); and

an electromechanical transducer (41, 42) that is configured to apply an acoustic wave (45) that propagates through the sample (20) held by the fluid holder (15), so as to separate, in the sample (20), a portion (20a) poor in particles and a portion (20b) rich in particles, the device being **characterized in that** the processor (32) is configured to:

> - identify, in the image, a region of interest corresponding to at least one portion (20a) poor in particles;
> - determine an optical property of the liquid medium, in the region of interest (20a) thus identified, the optical property varying depending on an amount of an analyte (26) in the sample; and
> - estimate the amount of the analyte (26) in the liquid medium (22), on the basis of said optical property in the region of interest.

**12.** Device according to Claim 11, wherein the electromechanical transducer (41, 42) includes a piezoelectric transducer or a plurality of piezoelectric transducers.

**13.** Device according to either one of Claims 11 and 12, wherein the electromechanical transducer applies a pressure to said fluid holder (15), so as to form an acoustic wave (45) in the sample (20) held by said fluid holder.

**14.** Device according to any one of Claims 11 to 13, wherein there are no magnifying optics between the image sensor (30) and the fluid holder (15).

**Fig. 1A**

**Fig. 1B**

**Fig. 1C**

**Fig. 1D**

**Fig. 2**

**Fig. 3A**

**Fig. 3B**

**Fig. 4A**                    **Fig. 4B**

**Fig. 5A**

**Fig. 5B**

**Fig. 6A**

**Fig. 6B**

**Fig. 6C**

**Fig. 7**

**Fig. 8**

**Fig. 9**

EP 3 220 131 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5866349 A
- EP 1875203 A
- WO 2011006525 A
- WO 9420833 A
- US 3964974 A